# EUROPEAN PATENT APPLICATION

(11) **EP 4 252 703 A1**
(43) Date of publication of application: **04.10.2023**
(21) Application number: 21901069.1
(22) Date of filing: 03.12.2021
(51) Int. Cl.: A61C 9/00, G16H 40/20, G16H 10/60, A61C 13/00, A61C 13/34, A61C 8/00, G06Q 30/06, G06Q 50/28, G06Q 50/22, G16H 30/00

(54) **METHOD, DEVICE AND RECORDING MEDIUM FOR DESIGNING, SELECTING, DELIVERING, OR IMPLANTING CUSTOMIZED READY-MADE ABUTMENT, OR PROVIDING INFORMATION RELATED THERETO**

(30) Priority: 04.12.2020 KR 20200169028
(71) Applicant: Osstemimplant Co., Ltd., Gangseo-gu Seoul 07789 (KR)
(72) Inventor: CHOI, Kyoo Ok, Seoul 07789 (KR); EOM, Tae Kwan, Seoul 07789 (KR); HEO, Jae Chan, Seoul 07789 (KR); LEE, Young Seok, Seoul 07789 (KR)
(74) Representative: Isarpatent
(86) International application number: PCT/KR2021/018230
(87) International publication number: WO 2022/119381

(57) **Abstract**

An embodiment provides a method for designing, selecting, delivering, or implanting a customized ready-made abutment, from multiple customized ready-made abutment sets designed or manufactured on the basis of a patient database, the method comprising the steps of: preparing multiple customized ready-made abutment sets on the basis of a patient database; picking up the oral data of a patient; selecting an appropriate abutment from the multiple customized ready-made abutment sets by using software on the basis of the picked-up oral data of the patient; and delivering or implanting the selected customized ready-made abutment.

## Description

### [Technical Field]

The present disclosure relates to a method, a device, and a recording medium for designing, selecting, delivering, or implanting a customized ready-made abutment or providing information related thereto.

### [Background Art]

An implant is a replacement material for restoring lost human tissue. In dentistry, an implant is generally a replacement material that is implanted and adhered to an alveolar bone from which a natural tooth root has become detached so as to replace the root of a lost tooth.

Such dental implants can be classified in various ways according to a method of combining an artificial tooth and a tooth root. Among the dental implants, there is a screw-type implant that uses a coupling force of a screw, and a conventional screw-type implant system (assembly) is composed of an implant, an abutment, an artificial tooth, and the like.

In order to perform implantation, an operator selects an appropriate implant or abutment from among products having various diameters and screw pitches in consideration of the patient's oral condition.

In particular, in an implant system, in the case of an abutment positioned between an implant implanted in an alveolar bone and an artificial tooth, various shapes of abutments may be used according to the condition of an implant implanted in a jawbone, the condition of the patient's oral cavity, or the like.

Recently, a method of manufacturing and using a customized abutment suitable for an individual patient using the computer-aided design (CAD) and computer-aided manufacturing (CAM) method without using an abutment provided as a ready-made product has been proposed.

Customized abutments take a long time to manufacture and are expensive because they are manufactured according to each individual. However, since the customized abutment is manufactured to fit an artificial tooth and a gingiva, it is possible to prevent food from being caught and it is aesthetically excellent. On the other hand, in the case of ready-made abutments, products that are pre-manufactured in large quantities may be received from manufacturers and used such that the ready-made abutments are mass-produced, and thus there is an advantage of being able to receive the ready-made abutments in a short period of time and being inexpensive. However, the ready-made abutment may not fit an artificial tooth and gingiva, and thus there is a problem that food may be caught or tooth dislocation may occur.

### [Disclosure]

### [Technical Problem]

The present disclosure is directed to providing a method, a device, and a recording medium for designing, selecting, delivering, or implanting a customized ready-made abutment or providing information related thereto. Specifically, the present disclosure is directed to providing a method, a device, and a recording medium for designing, selecting, delivering, or implanting a customized ready-made abutment having both an advantage of a customized abutment and an advantage of a ready-made abutment, or providing information related thereto.

The technical problem to be solved is not limited to the technical problems described above, and various technical problems may be further included within a range obvious to those skilled in the art.

### [Technical Solution]

One aspect of the present disclosure provides a method of designing, selecting, delivering, or implanting a customized ready-made abutment, from a plurality of customized ready-made abutment sets designed or manufactured on the basis of a patient database, which includes preparing for a plurality of customized ready-made abutment sets on the basis of a patient database; picking up oral data of a patient; selecting an appropriate abutment from among the plurality of customized ready-made abutment sets using software on the basis of the picked-up oral data of the patient; and delivering or implanting the selected customized ready-made abutment.

The number of the plurality of customized ready-made abutment sets prepared for based on the patient database may be 400 or more.

The preparing of the plurality of customized ready-made abutment sets on the basis of a patient database may include preparing for 400 or more customized ready-made abutment sets, dividing a statistical distribution range of patient-customized abutments into a plurality of regions on the basis of a unit length, and preparing for the 400 or more customized ready-made abutment sets on the basis of the plurality of region division results.

In the preparing of the 400 or more customized ready-made abutment sets on the basis of the plurality of region division results, the 400 or more customized ready-made abutment sets may be prepared for using 26 or more customized ready-made abutments classified according to horizontal and vertical lengths of an axial projection image of the customized ready-made abutment according to the plurality of region division results.

In the dividing of the statistical distribution range of the patient-customized abutments into the plurality of regions, grouping may be individually performed for each tooth type, the 400 or more customized ready-made abutment sets may be prepared for based on a result obtained by performing the grouping, and the tooth type may include at least one of a maxillary anterior tooth, a mandibular anterior tooth, a canine tooth, a premolar tooth, and a molar tooth.

In the selecting of the appropriate abutment, the appropriate abutment may be determined from among the plurality of customized ready-made abutment sets on the basis of size information about an implantation space of a target tooth.

The tooth type may include a maxillary anterior tooth, a mandibular anterior tooth, a canine tooth, a premolar tooth, and a molar tooth, and a selection of the type of tooth may be automatically recommended using software.

Further, a range of horizontal and vertical lengths of an axial projection image of the customized ready-made abutment may include at least one of 0.5 mm, 0.75 mm, and 1.0 mm.

Another aspect of the present disclosure provides a method of providing information related to a customized ready-made abutment, which includes determining a number of a plurality of customized ready-made abutments on the basis of a number of regions obtained as results obtained by dividing a statistical distribution range of the patient-customized abutments into a plurality of regions; obtaining oral data of a patient including size information about an implantation space of a target tooth; determining an appropriate abutment from among the plurality of customized ready-made abutment on the basis of the size information about the implantation space of the target tooth; and providing information about the appropriate abutment.

The number of the plurality of customized ready-made abutment sets prepared for based on the patient database may be 400 or more.

A type of the target tooth may include a maxillary anterior tooth, a mandibular anterior tooth, a canine tooth, a premolar tooth, and a molar tooth, and a selection of the tooth type may be automatically recommended using software.

The determining of the number of the plurality of customized ready-made abutments may include performing grouping on a statistical distribution range of abutment sizes for each tooth type on the basis of a unit length; and determining the number of the plurality of standard abutments to be 400 or more on the basis of a sum of numbers of groups obtained as results obtained by performing the grouping for each tooth type.

Still another aspect of the present disclosure provides a device for providing information related to a customized ready-made abutment, which includes a reception unit configured to acquire oral data of a patient including size information about an implantation space of a target tooth; and a processor configured to determine a number of the plurality of customized ready-made abutments to be 400 or more on the basis of a number of groups obtained as results obtained by dividing a statistical distribution range of the patient-customized abutments into a plurality of regions, determine an appropriate abutment from among the plurality of customized ready-made abutments on the basis of a type of the target tooth and the size information about the implantation space of the target tooth, and provide information about the appropriate abutment.

Yet another aspect of the present disclosure provides a computer-readable recording medium on which a program for executing the method according to any one of the first and second aspects on a computer is recorded. Alternatively, yet another aspect of the present disclosure provides a computer program stored in a recording medium to implement the method according to any one of the first and second aspects.

### [Advantageous Effects]

According to an embodiment, it is possible to provide a customized ready-made abutment that is cost-effective and has a high coverage for various patients.

Effects of the present disclosure are not limited to the above-described effects, and it should be understood that all possible effects deduced from a configuration of the present disclosure described in detailed descriptions and the appended claims are included.

### [Description of Drawings]

FIG. 1 is a diagram for describing a configuration of a customized ready-made abutment (100) according to an embodiment.
FIG. 2 is a diagram for describing a method of determining the number of customized ready-made abutments (100) according to a plurality of region division results according to an embodiment.
FIG. 3 is a schematic diagram illustrating an example of a configuration of a device (1000) according to an embodiment.
FIG. 4 is a flowchart illustrating a method for a device (1000) according to an embodiment to design, select, deliver, or implant a customized ready-made abutment (100), from a plurality of customized ready-made abutment sets designed or manufactured based on a patient database.
FIG. 5 is a diagram for describing a method of determining the number of customized ready-made abutments (100) according to results obtained by performing grouping for each tooth type on the basis of a height of a gingival part (120) according to an embodiment.
FIG. 6 is a diagram for describing a method of determining the number of customized ready-made abutments (100) according to results obtained by performing grouping for each tooth type on the basis of a height of a post (110) according to an embodiment.
FIG. 7 is a diagram for describing a method of determining the number of customized ready-made abutments (100) according to results obtained by performing grouping for each tooth type on the basis of an angle of a post (110) according to an embodiment.
FIGS. 8 to 16 are diagrams for describing various examples in which the number of customized ready-made abutments (100) is determined according to a result obtained by being divided a plurality of regions according to an embodiment.
FIG. 17 is a flowchart illustrating a method for a device (1000) according to another embodiment to provide information related to a customized ready-made abutment.

### [Modes of the Invention]

Although the terms used herein are selected from among general terms that are currently and widely used in consideration of functions in embodiments of the present disclosure, these may be changed according to intentions of those skilled in the art, precedents, or the advent of new technology. In addition, in a specific case, some terms may be arbitrarily selected by applicants. In this case, meanings thereof will be described in detail in a corresponding description of embodiments of the present disclosure. Therefore, the terms used herein should be defined based on meanings of the terms and content of this entire specification, rather than simply the terms themselves.

Throughout this specification, when a certain part "includes" a certain component, it means that another component may be further included not excluding another component unless otherwise defined. Moreover, terms described in the specification such as "...part," "...unit," "module," "device," refer to a unit of processing at least one function or operation, and may be implemented by hardware or software or a combination thereof.

Throughout this specification, the term "support" may be broadly interpreted in a sense that includes the performance of a related operation to achieve a specific purpose, and is not construed as limiting. In addition, throughout this specification, the term "providing" may be interpreted as including a process in which an object obtains specific information or directly or indirectly transmits/receives a specific object, and comprehensively including the performance of a related operation required in this process.

Hereinafter, embodiments of the present disclosure that can be easily performed by those skilled in the art will be described in detail with reference to the accompanying drawings. However, embodiments of the present disclosure may be implemented in several different forms, and are not limited to embodiments described herein.

Hereinafter, embodiments of the present disclosure will be described in detail with reference to the accompanying drawings.

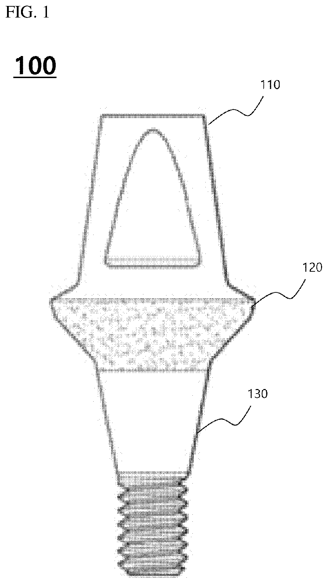

FIG. 1 is a diagram for describing a configuration of a customized ready-made abutment 100 according to an embodiment.

Referring to FIG. 1, the customized ready-made abutment 100 may include a post 110 positioned at an upper end thereof to be able to be coupled to an inside of a crown, a lower part 130 positioned at a lower end thereof such that a part or all of the lower part is able to be coupled to an implant, and a gingival part 120 positioned between the post 110 and the lower part 130 to be in contact with gingiva without being coupled to the implant.

The customized ready-made abutment 100 is a ready-made abutment having a customized abutment shape statistically optimized for oral environments of multiple patients.

A size of the customized ready-made abutment 100 may be determined according to various criteria. For example, the size of the customized ready-made abutment 100 may include horizontal and vertical lengths of an axial projection image of the abutment, a height of the gingival part 120, a height of the post 110, and the like.

The customized ready-made abutment 100 may be defined according to various criteria and measurement values thereof. For example, the customized ready-made abutment 100 may be defined using the horizontal and vertical lengths of the axial projection image of the abutment, the height of the gingival part 120, the height of the post 110, an angle of the post 110, and the like, but the present disclosure is not limited thereto. The angle of the post 110 may be an angle between the post 110 and the gingival part 120 or an angle between the post 110 and the lower part 130.

The size of the customized ready-made abutment 100 or the angle of the post 110 may be determined according to a region division result for an entire statistical distribution range of patient-customized abutments. Here, the region division may be interpreted as one or more partial distribution ranges specified on a two-dimensional distribution or a one-dimensional distribution of statistical data.

According to an embodiment, the region division may be performed based on a unit length, and a difference in length between groups may be expressed as a multiple of the unit length.

According to an embodiment, a range of the unit length may include at least one of 0.5 mm, 0.75 mm, and 1.0 mm. Specifically, a range of the horizontal and vertical lengths of the axial projection image of the customized ready-made abutment 100 may include at least one of 0.5 mm, 0.75 mm, and 1.0 mm. For example, the unit length may correspond to at least one of 0.5 mm, 0.75 mm, and 1.0 mm. In this case, when the unit length corresponds to 0.5 mm, the unit length may be a value within a preset range (e.g., 0.1 mm, 0.01 mm, 0.2mm, etc.) from 0.5 mm, when the unit length corresponds to 0.75 mm, the unit length may be a value within a preset range from 0.75 mm, and when the unit length corresponds to 1.0 mm, the unit length may be a value within a preset range from 1.0 mm, but the present disclosure is not limited thereto.

The region division may be performed for each tooth type, and the tooth type may include at least one of a maxillary anterior tooth, a mandibular anterior tooth, a canine tooth, a premolar tooth, and a molar tooth.

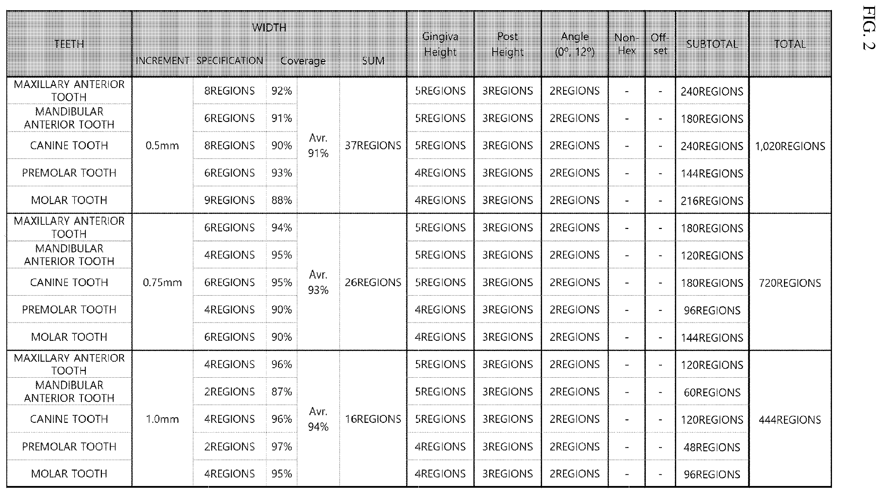

FIG. 2 is a diagram for describing a method of determining the number of customized ready-made abutments 100 according to a plurality of region division results according to an embodiment.

Referring to FIG. 2, according to an embodiment, a statistical distribution range of horizontal and vertical lengths of an axial projection image of the customized ready-made abutment 100 may be divided into a plurality of regions on the basis of a unit length corresponding to 1.0 mm. In this case, a maxillary anterior tooth may be divided into 4 regions (in this case, a coverage corresponds to 96%), a mandibular anterior tooth may be divided into 2 regions (in this case, a coverage corresponds to 87%), a canine tooth may be divided into 4 regions (in this case, a coverage corresponds to 96%), a premolar tooth may be divided into 2 regions (in this case, a coverage corresponds to 97%), and a molar tooth may be divided into 4 regions (in this case, a coverage corresponds to 95%). Therefore, 16 region division results for the horizontal and vertical lengths of the axial projection image of the customized ready-made abutment 100 may be obtained.

According to an embodiment, a statistical distribution range of a height of a gingival part 120 may be divided into a plurality of regions on the basis of the unit length corresponding to 1.0 mm. In this case, the maxillary anterior tooth may be divided into 5 regions, the mandibular anterior tooth may be divided into 5 regions, the canine tooth may be divided into 5 regions, the premolar tooth may be divided into 4 regions, and the molar tooth may be divided into 4 regions.

According to an embodiment, a statistical distribution range of a height of a post 110 may be divided into a plurality of regions on the basis of the unit length corresponding to 1.0 mm. In this case, the maxillary anterior tooth may be divided into 3 regions, the mandibular anterior tooth may be divided into 3 regions, the canine tooth may be divided into 3 regions, the premolar tooth may be divided into 3 regions, and the molar tooth may be divided into 3 regions.

According to an embodiment, a statistical distribution range of an angle of the post 110 may be divided into a plurality of regions on the basis of a unit angle corresponding to 12 degrees. In this case, the maxillary anterior tooth may be divided into 2 regions, the mandibular anterior tooth may be divided into 2 regions, the canine tooth may be divided into 2 regions, the premolar tooth may be divided into 2 regions (or 1 region), and the molar tooth may be divided into 2 regions (or 1 region).

Therefore, the maxillary anterior tooth may be divided into 4 regions with respect to the horizontal and vertical lengths of the axial projection image, 5 regions with respect to the height of the gingival part 120, 3 regions with respect to the height of the post 110, and 2 regions with respect to the angle of the post 110, and thus a total of 120 region division results may be obtained. Therefore, 120 customized ready-made abutments 100 may be determined for the maxillary anterior tooth. In a similar way, 60 region division results may be obtained for the mandibular anterior tooth, 120 region division results may be obtained for the canine tooth, 48 region division results may be obtained for the premolar tooth, and 96 region division results may be obtained for the molar tooth. Therefore, when the unit length corresponds to 1 mm and the unit angle corresponds to 12 degrees, 444 region division results may be obtained.

According to an embodiment, the statistical distribution range of the horizontal and vertical lengths of the axial projection image of the customized ready-made abutment 100 may be divided into a plurality of regions on the basis of a unit length corresponding to 0.75 mm. In this case, the maxillary anterior tooth may be divided into 6 regions (in this case, a coverage corresponds to 94%), the mandibular anterior tooth may be divided into 4 regions (in this case, a coverage corresponds to 95%), the canine tooth may be divided into 6 regions (in this case, a coverage corresponds to 95%), the premolar tooth may be divided into 4 regions (in this case, a coverage corresponds to 90%), and the molar tooth may be divided into 6 regions (in this case, a coverage corresponds to 90%). Therefore, 26 region division results for the horizontal and vertical lengths of the axial projection image of the customized ready-made abutment 100 may be obtained.

According to an embodiment, the statistical distribution range of the height of the gingival part 120 may be divided into a plurality of regions on the basis of the unit length corresponding to 0.75 mm. In this case, the maxillary anterior tooth may be divided into 5 regions, the mandibular anterior tooth may be divided into 5 regions, the canine tooth may be divided into 5 regions, the premolar tooth may be divided into 4 regions, and the molar tooth may be divided into 4 regions.

According to an embodiment, the statistical distribution range of the height of the post 110 may be divided into a plurality of regions on the basis of the unit length corresponding to 0.75 mm. In this case, the maxillary anterior tooth may be divided into 3 regions, the mandibular anterior tooth may be divided into 3 regions, the canine tooth may be divided into 3 regions, the premolar tooth may be divided into 3 regions, and the molar tooth may be divided into 3 regions.

According to an embodiment, the statistical distribution range of the angle of the post 110 may be divided into a plurality of regions on the basis of a unit angle corresponding to 12 degrees. In this case, the maxillary anterior tooth may be divided into 2 regions, the mandibular anterior tooth may be divided into 2 regions, the canine tooth may be divided into 2 regions, the premolar tooth may be divided into 2 regions (or 1 region), and the molar tooth may be divided into 2 regions (or 1 region).

Therefore, the maxillary anterior tooth may be divided into 6 regions with respect to the horizontal and vertical lengths of the axial projection image, 5 regions with respect to the height of the gingival part 120, 3 regions with respect to the height of the post 110, and 2 regions with respect to the angle of the post 110, and thus a total of 180 region division results may be obtained. Therefore, 180 customized ready-made abutments 100 may be determined for the maxillary anterior tooth. In a similar way, 120 region division results may be obtained for the mandibular anterior tooth, 180 region division results may be obtained for the canine tooth, 96 region division results may be obtained for the premolar tooth, and 144 region division results may be obtained for the molar tooth. Therefore, when the unit length corresponds to 0.75 mm and the unit angle corresponds to 12 degrees, 720 region division results may be obtained.

According to an embodiment, the statistical distribution range of the horizontal and vertical lengths of the axial projection image of the customized ready-made abutment 100 may be divided into a plurality of regions on the basis of a unit length corresponding to 0.5 mm. In this case, the maxillary anterior tooth may be divided into 8 regions (in this case, a coverage corresponds to 92%), the mandibular anterior tooth may be divided into 6 regions (in this case, a coverage corresponds to 91%), the canine tooth may be divided into 8 regions (in this case, a coverage corresponds to 90%), the premolar tooth may be divided into 6 regions (in this case, a coverage corresponds to 93%), and the molar tooth may be divided into 9 regions (in this case, a coverage corresponds to 88%). Therefore, 37 region division results for the horizontal and vertical lengths of the axial projection image of the customized ready-made abutment 100 may be obtained.

According to an embodiment, the statistical distribution range of the height of the gingival part 120 may be divided into a plurality of regions on the basis of the unit length corresponding to 0.5 mm. In this case, the maxillary anterior tooth may be divided into 5 regions, the mandibular anterior tooth may be divided into 5 regions, the canine tooth may be divided into 5 regions, the premolar tooth may be divided into 4 regions, and the molar tooth may be divided into 4 regions.

According to an embodiment, the statistical distribution range of the height of the post 110 may be divided into a plurality of regions on the basis of the unit length corresponding to 0.5 mm. In this case, the maxillary anterior tooth may be divided into 3 regions, the mandibular anterior tooth may be divided into 3 regions, the canine tooth may be divided into 3 regions, the premolar tooth may be divided into 3 regions, and the molar tooth may be divided into 3 regions.

According to an embodiment, the statistical distribution range of the angle of the post 110 may be divided into a plurality of regions on the basis of a unit angle corresponding to 12 degrees. In this case, the maxillary anterior tooth may be divided into 2 regions, the mandibular anterior tooth may be divided into 2 regions, the canine tooth may be divided into 2 regions, the premolar tooth may be divided into 2 regions (or 1 region), and the molar tooth may be divided into 2 regions (or 1 region).

Therefore, the maxillary anterior tooth may be divided into 8 regions with respect to the horizontal and vertical lengths of the axial projection image, 5 regions with respect to the height of the gingival part 120, 3 regions with respect to the height of the post 110, and 2 regions with respect to the angle of the post 110, and thus a total of 240 region division results may be obtained. Therefore, 240 customized ready-made abutments 100 may be determined for the maxillary anterior tooth. In a similar way, 180 region division results may be obtained for the mandibular anterior tooth, 240 region division results may be obtained for the canine tooth, 144 region division results may be obtained for the premolar tooth, and 216 region division results may be obtained for the molar tooth. Therefore, when the unit length corresponds to 0.5 mm and the unit angle corresponds to 12 degrees, 1,020 region division results may be obtained.

Referring to FIG. 2, when the unit length corresponds to 0.5 mm, 0.75 mm, or 1 mm and the unit angle corresponds to 12 degrees, 400 regions or more region division results may be obtained. As can be seen in all the examples of 0.5 mm, 0.75 mm, and 1 mm disclosed in FIG. 2, the number (or the number of customized ready-made abutments) of region division results may be maintained at 400 or more in all cases. Even when the customized abutment is a ready-made abutment, the effect of the customized abutment can be expected only when the number (or the number of customized ready-made abutments) of region division results is greater than or equal to a certain value. According to the present disclosure, in various examples, the number (or the number of customized ready-made abutments) of region division results of 400 or more may be maintained, and thus the effect of a customized abutment can be expected even when the customized abutment is a ready-made abutment.

A minimum lower limit is required to utilize a ready-made abutment as a customized abutment, and the effect of the customized abutment can be expected by maintaining a lower limit of 400 as shown in FIG. 2.

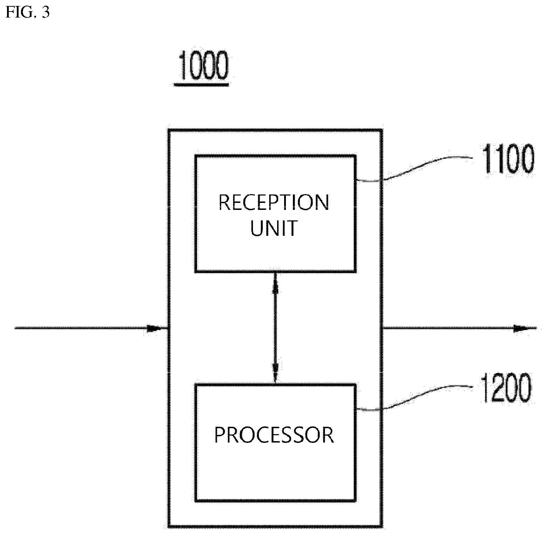

FIG. 3 is a schematic diagram illustrating an example of a configuration of a device 1000 according to an embodiment.

The device 1000 according to an embodiment may include a reception unit 1100 that acquires 400 or more customized ready-made abutment sets on the basis of a patient database, and a processor 1200 that picks up patient's oral data, selects an appropriate abutment from the plurality of customized ready-made abutment sets using software on the basis of the patient's oral data that has been picked up, and supports delivery or implantation of the selected customized ready-made abutment.

The device 1000 according to another embodiment may include a reception unit 1100 that acquires patient's oral data including size information about an implantation space of a target tooth, and a processor 1200 that determines the number of customized ready-made abutments to be 400 or more on the basis of the number of regions obtained as results obtained by dividing a statistical distribution range of patient-customized abutments into a plurality of regions, determines an appropriate abutment from among the plurality of customized ready-made abutments on the basis of the type of the target tooth and/or the size information about the implantation space of the target tooth, and provides information about the appropriate abutment. The tooth type may include a maxillary anterior tooth, a mandibular anterior tooth, a canine tooth, a premolar tooth, and a molar tooth, but the present disclosure is not limited thereto. Further, a selection of the tooth type may be performed by an automatic recommendation method using software. For example, the device 1000 may provide recommendation information about any one of a maxillary anterior tooth, a mandibular anterior tooth, a canine tooth, a premolar tooth, and a molar tooth using the patient's oral data including the size information about the implantation space of the target tooth. Further, the tooth type may be selected based on a user input for the recommendation information. The term "selection" may be interpreted as including determination, but the present disclosure is not limited thereto.

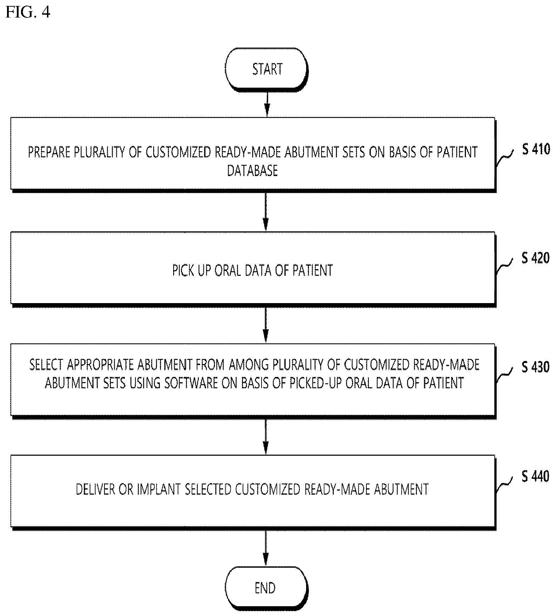

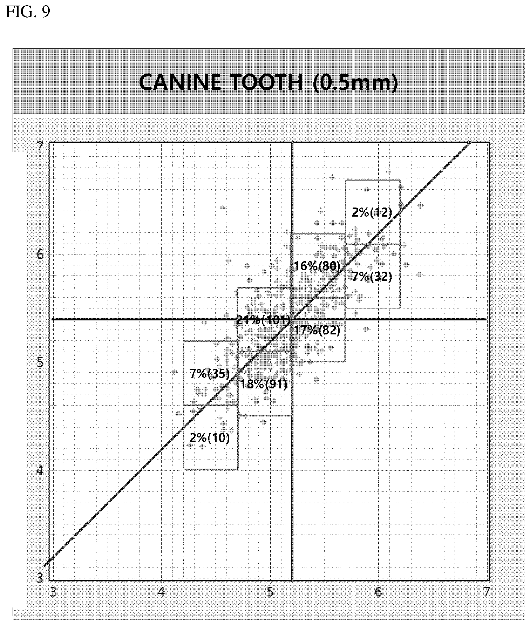

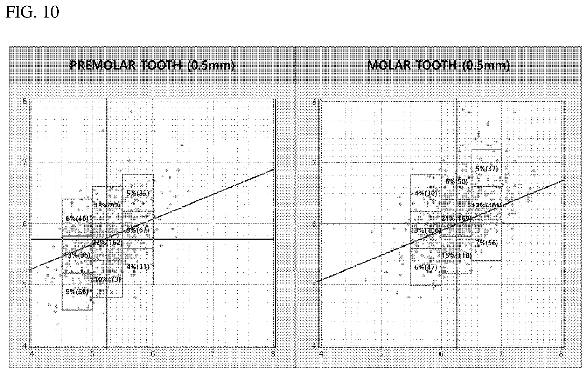

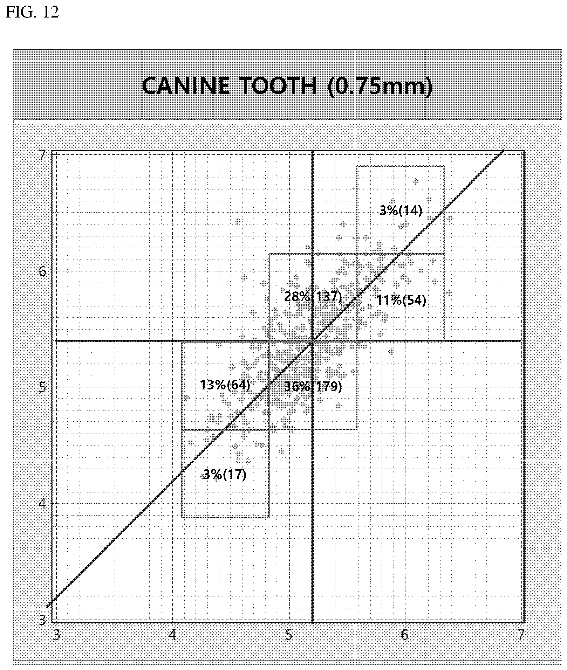

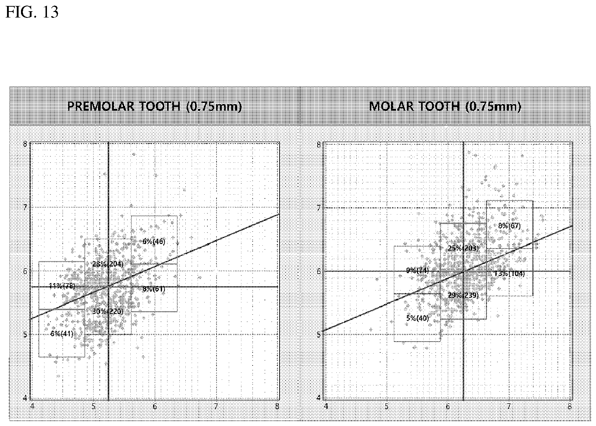

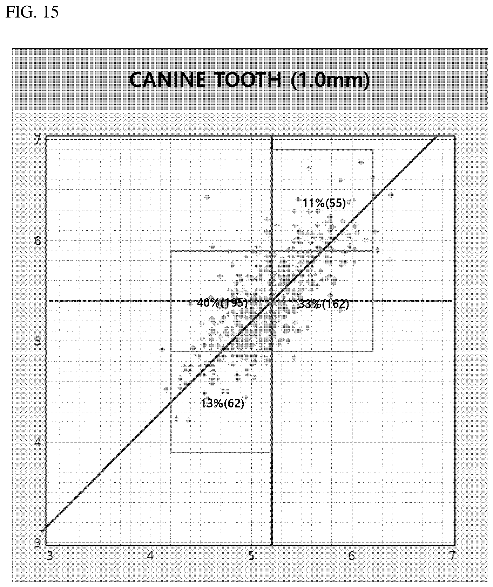

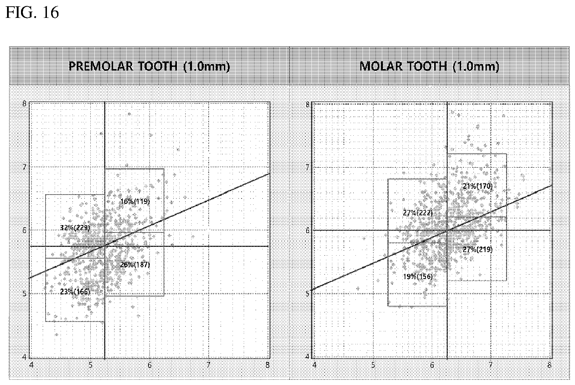

A series of operations according to another embodiment described above will be described below with reference to FIGS. 4 to 17.

The reception unit 1100 according to an embodiment may receive pieces of information described throughout this specification from another device, a patient database, or a memory, and may include, for example, a wired/wireless communication device that can transmit or receives various pieces of information described below by being connected to another device through a network. Further, the processor 1200 may perform a series of operations to design, select, deliver, or implant a customized ready-made abutment or provide information related to the customized ready-made abutment, may be implemented as a central processor unit (CPU) that controls the overall operation of the device 1000, and may be electrically connected to the reception unit 1100 and other components to control data flow therebetween.

Further, those skilled in the art may understand that other general-purpose components other than the components illustrated in FIG. 3 may be further included in the device 1000. For example, the device 1000 may further include a patient database for storing patient data, a memory for storing data used throughout operations, an input/output interface for receiving user input or outputting information, and the like.

FIG. 4 is a flowchart illustrating a method for a device 1000 according to an embodiment to design, select, deliver, or implant a customized ready-made abutment 100, from a plurality of customized ready-made abutment sets designed or manufactured based on a patient database.

Referring to FIG. 4, in operation S410, the device 1000 may prepare for a plurality of customized ready-made abutment sets on the basis of a patient database. In an embodiment, the device 1000 may collect oral data for a plurality of patients to build a patient database including the oral data, or receive the oral data for the plurality of patients from a pre-built patient database, and may analyze a statistical distribution of abutment data for each patient using the oral data for the plurality of patients and determine the plurality of customized ready-made abutment sets by performing region division according to a result of the analysis. For example, the device 1000 may determine 400 or more customized ready-made abutment sets by being divided a plurality of regions on the basis of the statistical population distribution.

In operation S420, the device 1000 may pick up the patient's oral data. In an embodiment, the device 1000 may generate the patient's oral data including size information about an implantation space of a target tooth through imaging, receive the patient's oral data from a user device, or receive the patient's oral data by a user input. In an embodiment, the patient's oral data may include three-dimensional (3D) surface scan data.

In operation S430, the device 1000 may select an appropriate abutment from among the plurality of customized ready-made abutment sets using software on the basis of the patient's oral data that has been picked up. For example, the device 1000 may determine size information about a patient-customized abutment suitable for the patient from the patient's oral data, or determine an appropriate one from among the 400 or more customized ready-made abutment sets obtained as the region division results using the size information about the implantation space of the target tooth included in the oral data.

In an embodiment, the device 1000 may determine an appropriate abutment from among the plurality of customized ready-made abutment sets on the basis of the type of target tooth and/or the size information about the implantation space of the target tooth. For example, the device 1000 may determine a type (e.g., a maxillary anterior tooth) of target tooth for which an implantation is required for the patient, a width (e.g., 0.75 mm) of the implantation space of the target tooth, a height of the gingival part 120, a height of the post 110, an angle (e.g., 12 degrees) of the post 110, and the like, from the patient's oral data, and may select one corresponding thereto from among the 400 or more customized ready-made abutment sets as an abutment optimized for the patient.

In an embodiment, the device 1000 may obtain a virtual placement result through a simulation of implanting the determined customized ready-made abutment sets on the patient's oral data, and accordingly, determine whether a corresponding customized ready-made abutment 100 fits the patient.

In operation S440, the device 1000 may deliver or implant the selected customized ready-made abutment 100. For example, when it is determined that the abutment fits the patient well, the device 1000 may provide order information about the selected customized ready-made abutment 100 to support delivery or implantation of the customized ready-made abutment 100. Further, upon receiving a user's order request for the provided order information, the device 1000 may support delivery of a corresponding customized ready-made abutment 100 that is pre-manufactured, and accordingly, the customized ready-made abutment 100 delivered to the user may be used in the implantation process for the patient.

In an embodiment, when there are a plurality of selected customized ready-made abutments 100, the device 1000 may provide information about a plurality of selected customized ready-made abutments according to priority, and deliver or implant the customized ready-made abutment 100 that is finally selected based on a selection input for any one of the plurality of customized ready-made abutments 100. For example, the device 1000 may display an abutment recommendation list including the information about the plurality of selected customized ready-made abutments 100, and finally select the customized ready-made abutment 100 according to the user's selection input therefor.

In an embodiment, when there are a plurality of selected customized ready-made abutments 100, different priorities may be assigned to the height of the gingival part 120, the height of the post 110, and the angle of the post 110, and for example, as being close to a statistical average value for the customized ready-made abutment sets, higher priority may be assigned to the closest target in the order of the height of the gingival part 120, the height of the post 110, and the angle of the post 110.

In an embodiment, the device 1000 may obtain the oral data for the plurality of patients and the abutment data for the patient-customized abutment. For example, the device 1000 may collect the patient's oral data, and derive the abutment data including the size information of the patient-customized abutment suitable for the type of target tooth for each patient by measuring and analyzing the patient's oral data, and for another example, the device 1000 may collect the patient's oral data and collect the data for the abutment that has been implanted into the patient or derived to be customized for the patient according to measurement and analysis.

In an embodiment, the oral data may be measured by conventional impression taking methods or scans, X-rays, computed tomography (CT), etc., and may include at least one of a type and position of a tooth, a height of gingiva, a shape of the gingiva, an angle between the tooth and a bone, a size of the tooth, and a diameter of the tooth. In an embodiment, the abutment data may include at least one of a type of the patient-customized abutment, size information about the patient-customized abutment, and a tooth type corresponding thereto. In an embodiment, the oral data and the abutment data may be linked to each other for each patient.

In an embodiment, the device 1000 may determine one or more parameters on the basis of measured and analyzed data of the patient-customized abutment, for example, the device 1000 may analyze the abutment data matched with the oral data to select the parameters, and for another example, the device 1000 may receive a part considered important by the user by a user input to select the part as a parameter. In an embodiment, one or more parameters may include values of the various elements described above, such as the heights of the post 110, the gingival part 120, and the lower part 130 of the customized ready-made abutment 100, the horizontal and vertical lengths of the axial projection image, the angle, etc., and one or more specific parameters may be selected from among the above elements according to a predetermined priority or a user input.

In an embodiment, the device 1000 may obtain a statistical distribution of patient-customized abutments on the basis of one or more parameters, and for example, may measure and analyze the statistical distribution of the patient-customized abutments on the basis of the selected parameters.

In an embodiment, the device 1000 may divide a statistical distribution range of the patient-customized abutments into a plurality of regions on the basis of the unit length, and the device 1000 may prepare for 400 or more customized ready-made abutment sets on the basis of a plurality of region division results. For example, as described above with reference to FIGS. 1 and 2, the device 1000 may divide the tooth into the plurality of regions in a method in which an axial projection image of each patient-customized abutment is obtained, horizontal and vertical lengths of the abutment on the projection image is obtained, a statistical distribution (e.g., normal distribution) when the horizontal and vertical lengths are incremented by a preset unit length (e.g., 0.75 mm), and intervals that satisfy preset conditions (e.g., 70% or more) within the statistical distribution are classified into a preset number (e.g., 5), and the device 1000 may prepare for 400 or more customized ready-made abutment sets using 26 or more customized ready-made abutments 100 classified according to the horizontal and vertical lengths of the axial projection image of the abutment according to the region division results.

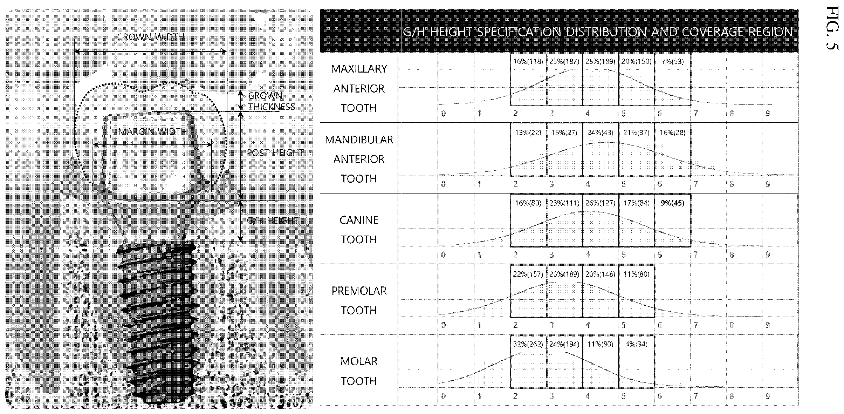

In an embodiment, the device 1000 may individually perform grouping for each tooth type, and the tooth type may include at least one of a maxillary anterior tooth, a mandibular anterior tooth, a canine tooth, a premolar tooth, and a molar tooth. Further, the device 1000 may individually perform grouping on each of the plurality of parameters, and the plurality of parameters may include at least one of the height of the gingival part 120, the height of the post 110, and the angle of the post 110 as described above. Content thereof will be described with reference to FIGS. 5 to 7.

FIG. 5 is a diagram for describing a method of determining the number of customized ready-made abutments 100 according to results obtained by performing grouping for each tooth type on the basis of a height of a gingival part 120 according to an embodiment.

Referring to FIG. 5, according to an embodiment, a statistical distribution range of a height (G/H) of the gingival part 120 of the customized ready-made abutment 100 may be divided into a plurality of regions on the basis of a preset unit length (e.g., 1.0 mm), and the region division results may be grouped according to a group of each tooth type. In this case, a maxillary anterior tooth may be grouped into 6 groups (in this case, a coverage corresponds to 99%), a mandibular anterior tooth may be grouped into 6 groups (in this case, a coverage corresponds to 98%), a canine tooth may be grouped into 6 groups (in this case, a coverage corresponds to 99%), a premolar tooth may be grouped into 6 groups (in this case, a coverage corresponds to 97%), and a molar tooth may be grouped into 6 groups (in this case, a coverage corresponds to 95%).

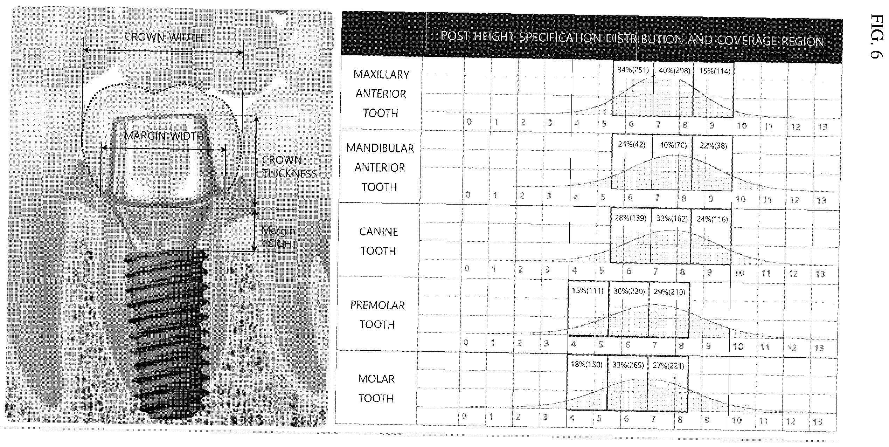

FIG. 6 is a diagram for describing a method of determining the number of customized ready-made abutments 100 according to results obtained by performing grouping for each tooth type on the basis of a height of a post 110 according to an embodiment.

Referring to FIG. 6, according to an embodiment, a statistical distribution range of the height of the post 110 of the customized ready-made abutment 100 may be divided into a plurality of regions on the basis of a preset unit length (e.g., 1.0 mm), and the region division results may be grouped according to a group of each tooth type. In this case, a maxillary anterior tooth may be grouped into 3 groups (in this case, a coverage corresponds to 89%), a mandibular anterior tooth may be grouped into 3 groups (in this case, a coverage corresponds to 85%), a canine tooth may be grouped into 3 groups (in this case, a coverage corresponds to 85%), a premolar tooth may be grouped into 3 groups (in this case, a coverage corresponds to 75%), and the molar tooth may be grouped into 3 groups (in this case, a coverage corresponds to 78%).

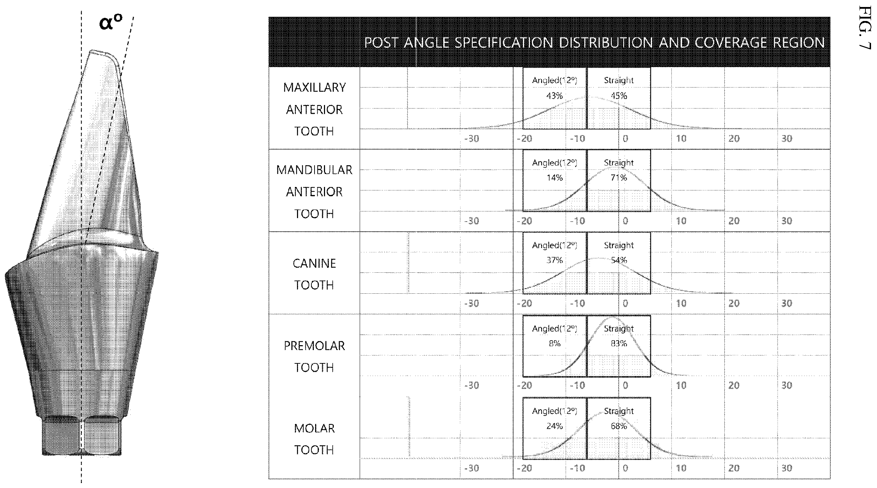

FIG. 7 is a diagram for describing a method of determining the number of customized ready-made abutments 100 according to results obtained by performing grouping for each tooth type on the basis of an angle of a post 110 according to an embodiment.

Referring to FIG. 7, according to an embodiment, a statistical distribution range of the angle of the post 110 of the customized ready-made abutment 100 may be divided into a plurality of regions on the basis of a preset unit angle (e.g., 12 degrees), and the region division results may be grouped according to a group of each tooth type. In this case, a maxillary anterior tooth may be grouped into 2 groups (in this case, a coverage corresponds to 88%), a mandibular anterior tooth may be grouped into 2 groups (in this case, a coverage corresponds to 85%), a canine tooth may be grouped into 2 groups (in this case, a coverage corresponds to 91%), a premolar tooth may be grouped into 1 group (in this case, a coverage corresponds to 83%), and a molar tooth may be grouped into 1 group (in this case, a coverage corresponds to 68%).

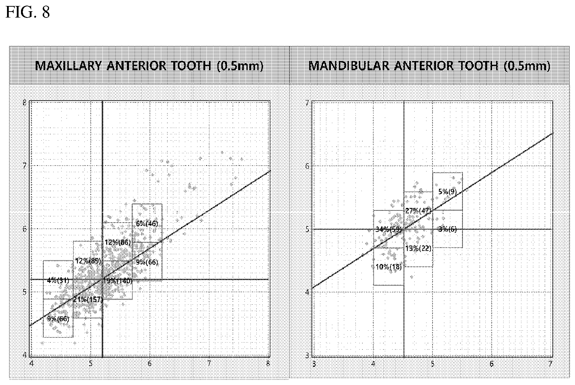

FIGS. 8 to 16 are diagrams for describing various examples in which the number of customized ready-made abutments 100 is determined according to a result obtained by being divided a plurality of regions according to an embodiment.

Referring to FIGS. 8 to 10, a statistical distribution range of horizontal and vertical lengths of an axial projection image of the customized ready-made abutment 100 may be divided into a plurality of regions on the basis of a unit length corresponding to 0.5 mm. In this case, a maxillary anterior tooth may be divided into 8 regions and thus a coverage of 92% may be secured, a mandibular anterior tooth may be divided into 6 regions and thus a coverage of 91% may be secured, a canine tooth may be divided into 8 regions and thus a coverage of 90% may be secured, a premolar tooth may be divided into 9 regions and thus a coverage of 93% may be secured, and a molar tooth may be divided into 8 regions and thus a coverage of 88% may be secured.

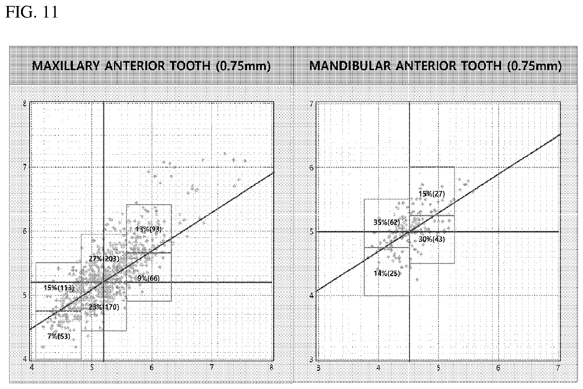

Referring to FIGS. 11 to 13, according to an embodiment, a statistical distribution range of horizontal and vertical lengths of an axial projection image of a customized ready-made abutment 100 may be divided into a plurality of regions on the basis of a unit length corresponding to 0.75 mm. In this case, a maxillary anterior tooth may be divided into 6 regions to secure a coverage of 94%, a mandibular anterior tooth may be divided into 4 regions to secure a coverage of 95%, a canine tooth may be divided into 6 regions to secure a coverage of 95%, a premolar tooth may be divided into 6 regions to secure a coverage of 90%, and a molar tooth may be divided into 6 regions to secure a coverage of 90%.

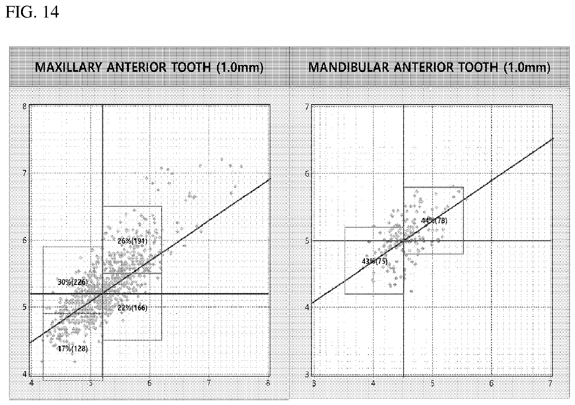

Referring to FIGS. 14 to 16, according to an embodiment, a statistical distribution range of horizontal and vertical lengths of an axial projection image of a customized ready-made abutment 100 may be divided into a plurality of regions on the basis of a unit length corresponding to 1.0 mm. In this case, a maxillary anterior tooth may be divided into 4 regions to secure a coverage of 96%, a mandibular anterior tooth may be divided into 2 regions to secure a coverage of 87%, a canine tooth may be divided into 4 regions to secure a coverage of 96%, a premolar tooth may be divided into 4 regions to secure a coverage of 97%, and a molar tooth may be divided into 4 regions to secure a coverage of 95%.

As described above, it can be confirmed that a high coverage of the entire statistical distribution range can be secured at a level of about 90% by appropriately setting the number of regions for each tooth type.

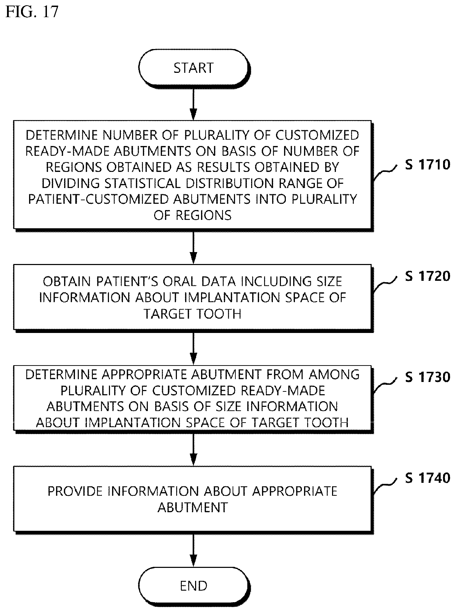

FIG. 17 is a flowchart illustrating a method for a device 1000 according to another embodiment to provide information related to a customized ready-made abutment.

Referring to FIG. 17, in operation S1710, the device 1000 may determine the number of a plurality of customized ready-made abutments on the basis of the number of regions obtained as results obtained by dividing a statistical distribution range of patient-customized abutments into a plurality of regions. In an embodiment, when a unit length of horizontal and vertical lengths of an axial projection image of the customized ready-made abutment 100 corresponds to 0.5 mm, 1,020 region division results may be obtained according to the region division results for the height of the gingival part 120, the height of the post 110, the angle of the post 110, etc., when the unit length corresponds to 0.75 mm, 720 region division results may be obtained, and when the unit length corresponds to 1.0 mm, 444 region division results may be obtained. Referring to various examples, the number of regions obtained as the region division results may be 400 or more.

In an embodiment, the device 1000 may perform grouping on a statistical distribution range of abutment sizes for each tooth type on the basis of the unit length, and determine the number of plurality of standard abutments to be 400 or more on the basis of the sum of the numbers of groups obtained as results obtained by performing the grouping for each tooth type. In an embodiment, the tooth type may include at least one of a maxillary anterior tooth, a mandibular anterior tooth, a canine tooth, a premolar tooth, and a molar tooth.

In operation S1720, the device 1000 may obtain patient's oral data including size information about an implantation space of a target tooth. In an embodiment, the device 1000 may receive patient's oral data further including a type of target tooth and size information about an implantation space of the target tooth from another device by a user input.

In operation S1730, the device 1000 may determine an appropriate abutment from among the plurality of customized ready-made abutments on the basis of the size information about the implantation space of the target tooth. For example, the device 1000 may determine, from the patient's oral data, a type (e.g., a maxillary anterior tooth) of target tooth for which an implantation is required for the patient, a width (e.g., 0.75 mm) of the implantation space of the target tooth, a height of the gingival part 120, a height of the post 110, an angle (e.g., 12 degrees) of the post 110, and the like, from the patient's oral data, and may select one corresponding thereto from among the 400 or more customized ready-made abutment sets as an abutment optimized for the patient.

In operation S1740, the device 1000 may provide information about the appropriate abutment, and for example, and display optimal abutment information including detailed information about an abutment type of the finally-selected customized ready-made abutment 100, the type (e.g., the maxillary anterior tooth) of target tooth, the width (e.g., 0.75 mm) of the implantation space of the target tooth, the height of the gingival part 120, the height of the post 110, the angle (e.g., 12 degrees) of the post 110, and the like or transmit the optimal abutment information to the user device.

In an embodiment, when there are a plurality of selected customized ready-made abutments 100, the device 1000 may provide information about a plurality of selected customized ready-made abutments according to priority, and provide information about the customized ready-made abutment 100 that is finally selected based on a selection input for any one of the plurality of customized ready-made abutments 100.

According to an embodiment of the present disclosure, it is possible to rapidly provide a dental abutment that is cost-effective and aesthetically superior to a customer.

Although specific numerical values are illustrated throughout the drawings, this is not limited thereto as an embodiment, and may be implemented by including them or being variously modified, and some of the above-described operations may be implemented in various modified forms in terms of order, function, and branching.

Meanwhile, the above-described method may be written with a program that can be executed on a computer, and may be implemented in a general-purpose digital computer that operates the program using a computer-readable recording medium. Further, a structure of data used in the above-described method may be recorded on a computer-readable recording medium through various means. The computer-readable recording medium includes storage media such as magnetic storage media (e.g., a read only memory (ROM), a random-access memory (RAM), a Universal Serial Bus (USB), a floppy disk, a hard disk, etc.) and optical reading media (e.g., a compact disc read only memory (CD-ROM), a digital versatile disc (DVD), etc.).

It will be understood by those skilled in the art that various changes may be made without departing from the spirit and scope of the present disclosure. Therefore, the disclosed methods should be considered in a descriptive sense only and not for purposes of limitation. The scope of the present disclosure is defined not by the above description but by the appended claims, and encompasses all modifications and equivalents that fall within the scope of the appended claims and will be construed as being included in the present disclosure.

## Claims

1. A method of designing, selecting, delivering, or implanting a customized ready-made abutment, from a plurality of customized ready-made abutment sets designed or manufactured based on a patient database, the method comprising:
preparing for a plurality of customized ready-made abutment sets on the basis of a patient database;
picking up oral data of a patient;
selecting an appropriate abutment from among the plurality of customized ready-made abutment sets using software on the basis of the picked-up oral data of the patient; and
delivering or implanting the selected customized ready-made abutment.

2. The method of claim 1, wherein a number of the plurality of customized ready-made abutment sets prepared for based on the patient database is 400 or more.

3. The method of claim 1, the preparing of the plurality of customized ready-made abutment sets on the basis of a patient database includes:
preparing for 400 or more customized ready-made abutment sets;
dividing a statistical distribution range of patient-customized abutments into a plurality of regions on the basis of a unit length; and
preparing for the 400 or more customized ready-made abutment sets on the basis of the plurality of region division results.

4. The method of claim 3, wherein, in the preparing of the 400 or more customized ready-made abutment sets on the basis of the plurality of region division results, the 400 or more customized ready-made abutment sets are prepared for using 26 or more customized ready-made abutments classified according to horizontal and vertical lengths of an axial projection image of the customized ready-made abutment according to the plurality of region division results.

5. The method of claim 3, wherein, in the dividing of the statistical distribution range of the patient-customized abutments into the plurality of regions, grouping is individually performed for each tooth type, the 400 or more customized ready-made abutment sets are prepared for based on a result obtained by performing the grouping, and the tooth type includes at least one of a maxillary anterior tooth, a mandibular anterior tooth, a canine tooth, a premolar tooth, and a molar tooth.

6. The method of claim 1, wherein, in the selecting of the appropriate abutment, the appropriate abutment is determined from among the plurality of customized ready-made abutment sets on the basis of size information about an implantation space of a target tooth.

7. The method of claim 5, wherein the tooth type includes a maxillary anterior tooth, a mandibular anterior tooth, a canine tooth, a premolar tooth, and a molar tooth, and a selection of the type of tooth is automatically recommended using software.

8. The method of claim 3, wherein a range of horizontal and vertical lengths of an axial projection image of the customized ready-made abutment includes at least one of 0.5 mm, 0.75 mm, and 1.0 mm.

9. A method of providing information related to a customized ready-made abutment, the method comprising:
determining a number of a plurality of customized ready-made abutments on the basis of a number of regions obtained as results obtained by dividing a statistical distribution range of the patient-customized abutments into a plurality of regions;
obtaining oral data of a patient including size information about an implantation space of a target tooth;
determining an appropriate abutment from among the plurality of customized ready-made abutment on the basis of the size information about the implantation space of the target tooth; and
providing information about the appropriate abutment.

10. The method of claim 9, wherein a number of the plurality of customized ready-made abutment sets prepared for based on the patient database is 400 or more.

11. The method of claim 9, wherein a type of the target tooth includes a maxillary anterior tooth, a mandibular anterior tooth, a canine tooth, a premolar tooth, and a molar tooth, and a selection of the tooth type is automatically recommended using software.

12. The method of claim 9, wherein the determining of the number of the plurality of customized ready-made abutments includes:
performing grouping on a statistical distribution range of abutment sizes for each tooth type on the basis of a unit length; and
determining the number of the plurality of standard abutments to be 400 or more on the basis of a sum of numbers of groups obtained as results obtained by performing the grouping for each tooth type.

13. A device for providing information related to a customized ready-made abutment, the device comprising:
a reception unit configured to acquire oral data of a patient including size information about an implantation space of a target tooth; and
a processor configured to determine a number of the plurality of customized ready-made abutments to be 400 or more on the basis of a number of groups obtained as results obtained by dividing a statistical distribution range of the patient-customized abutments into a plurality of regions, determine an appropriate abutment from among the plurality of customized ready-made abutments on the basis of a type of the target tooth and the size information about the implantation space of the target tooth, and provide information about the appropriate abutment.

14. A computer-readable recording medium on which a program for executing the method of claim 1 on a computer is recorded.
